# EUROPEAN PATENT SPECIFICATION

(11) **EP 4 085 853 B1**
(45) Date of publication and mention of the grant of the patent: **24.07.2024**
(21) Application number: 22181102.9
(22) Date of filing: 31.07.2018
(51) Int. Cl.: A61B 17/34, A61F 2/24, A61M 25/06, A61M 25/00

(54) **INTRODUCER SYSTEM WITH EXPANDABLE CAPABILITIES**
EINFÜHRSYSTEM MIT ERWEITERBAREN FÄHIGKEITEN
SYSTÈME D'INTRODUCTION AVEC CAPACITÉS D'EXTENSION

(30) Priority: 31.07.2017 US 201762539293 P
(43) Date of publication of application: 09.11.2022
(62) Divisional of application: 18755620.4
(73) Proprietor: Boston Scientific Scimed, Inc., Maple Grove, MN 55311 (US)
(72) Inventor: FITTERER, Mimi Trinh, Belmont, 94002 (US); INO, Takashi H., San Jose, 95123 (US)
(74) Representative: Peterreins Schley

(56) References cited:
- WO-A1-2016/164082
- US-A1- 2014 236 123
- US-A1- 2016 074 067
- US-A1- 2016 296 730
- US-A1- 2017 209 133

## Description

### TECHNICAL FIELD

The disclosure relates generally to medical devices and more particularly to medical devices that are adapted for use in percutaneous medical procedures.

### BACKGROUND

In some instances, performing percutaneous medical procedures may require the insertion and/or maneuvering of relatively large medical devices through a patient's vasculature. However, inserting the medical device into the vasculature may result in undesirable forces being applied to the vessel walls or at the aortotomy during tracking. For example, as the medical device passes into the vasculature, it may make undesirable contact with one or more vessel walls. This interference may cause injury to the vessel as the medical device is navigated into calcified or diseased vessels. Therefore, in some instances an introducer system is utilized to position an introducer sheath into the vessel, whereby the introducer sheath is utilized to facilitate the insertion of medical devices into the vessel. Vessel trauma resulting from forces applied to the vessel wall by a medical device may be lessened by minimizing the size of the introducer system used to access the vessel and provide a prototective barrier from the delivery system at the aortotomy. Therefore, it may be desirable to design an introducer system having a reduced insertion profile, whether inserted distally or aligned towards the back end of the delivery system. The implant delivery system may be removed in conjunction with or independently of the introducer sheath, depending on post procedures that would necessitate use of an introducer system.

WO 2016/164082 A1 discloses an implant delivery system having an expandable introducer sheath.

### SUMMARY

The invention is defined by claim 1. Further embodiments of the invention are defined by the dependent claims. Methods per se are not claimed.

This disclosure provides design, material, manufacturing method, and use alternatives for medical devices. An example implant delivery system includes a catheter shaft having a distal end region and proximal end region, an implant coupled to the distal end region of the catheter shaft, a handle coupled to the proximal end region of the catheter shaft and an introducer sheath disposed along the catheter shaft. The introducer sheath includes an inner liner including a lumen and an expandable support member. The support member includes a plurality of ribs extending along a length of the support member. The introducer sheath further includes a sheath attached to at least a portion of the support member, wherein the support member is designed to shift from a first position to an expanded position.

Alternatively or additionally to any of the examples above, wherein the introducer sheath is slidable along the catheter shaft.

Alternatively or additionally to any of the examples above, wherein the distal end region is designed to puncture into a body lumen.

Alternatively or additionally to any of the examples above, wherein the introducer sheath is unexpanded in the first position.

Alternatively or additionally to any of the examples above, wherein the plurality of rib members extend along only a portion of the introducer sheath.

Alternatively or additionally to any of the examples above, wherein the introducer sheath shifts to the expanded position as the proximal end region of the catheter shaft is withdrawn through the introducer sheath.

Alternatively or additionally to any of the examples above, wherein the expandable support member extends around at least a portion of the inner liner.

Alternatively or additionally to any of the examples above, wherein the support member is positioned between the inner liner and the sheath.

Alternatively or additionally to any of the examples above, wherein the plurality of ribs are free to move relative to the liner, the sheath, or both the liner and the sheath.

Alternatively or additionally to any of the examples above, wherein the plurality of ribs includes a first set of rib members and a second set of rib members, and wherein the first set of rib members interdigitate with the second set of rib members.

Another implant delivery system includes:
a catheter shaft having a distal end region and proximal end region;
an implantable heart valve releasably coupled to the distal end region of the catheter shaft;
a handle coupled to the proximal end region of the catheter shaft; and
an introducer sheath disposed along the catheter shaft and positioned proximal of the heart valve, wherein the introducer sheath includes:
   an inner liner including a lumen;
   an expandable support member, the support member including a plurality of ribs extending along a length of the support member; and
   a sheath attached to at least a portion of the support member;
   wherein the support member is designed to shift between an unexpanded configuration and an expanded configuration.

Alternatively or additionally to any of the examples above, wherein the introducer sheath is slidable along the catheter shaft.

Alternatively or additionally to any of the examples above, wherein the distal end region of the catheter shaft is designed to puncture into a body lumen.

Alternatively or additionally to any of the examples above, wherein the plurality of rib members extend along only a portion of the introducer sheath.

Alternatively or additionally to any of the examples above, wherein the introducer sheath shifts to the expanded position as the proximal end region of the catheter shaft is withdrawn through the introducer sheath.

Alternatively or additionally to any of the examples above, wherein the expandable support member extends around at least a portion of the inner liner.

Alternatively or additionally to any of the examples above, wherein the support member is positioned between the inner liner and the sheath.

Alternatively or additionally to any of the examples above, wherein the plurality of ribs are free to move relative to the liner, the sheath, or both the liner and the sheath.

Alternatively or additionally to any of the examples above, wherein the plurality of ribs includes a first set of rib members and a second set of rib members, and wherein the first set of rib members interdigitate with the second set of rib members.

Another implant delivery system includes:
a catheter shaft having a distal end region and proximal end region;
an implantable heart valve releasably coupled to the distal end region of the catheter shaft;
a handle coupled to the proximal end region of the catheter shaft; and
an introducer sheath disposed along the catheter shaft and positioned proximal of the heart valve, wherein the introducer sheath includes:
   an inner liner including a lumen;
   an expandable support member, the support member including a plurality of ribs extending along a length of the support member;
   a sheath attached to at least a portion of the support member;
   wherein the support member is designed to shift between an unexpanded configuration and an expanded configuration; and
   a proximal hub including an anti-splash back valve to prevent blood loss if the introducer sheath were to remain inside the body for additional procedures following implantation and/or removal of the implant delivery system.

Alternatively or additionally to any of the examples above, wherein the introducer sheath is slidable along the catheter shaft.

Alternatively or additionally to any of the examples above, wherein the distal end region of the catheter shaft is designed to puncture into a body lumen.

Alternatively or additionally to any of the examples above, wherein the plurality of rib members extend along only a portion of the introducer sheath.

Alternatively or additionally to any of the examples above, wherein the introducer sheath shifts to the expanded position as the proximal end region of the catheter shaft is withdrawn through the introducer sheath.

Alternatively or additionally to any of the examples above, wherein the expandable support member extends around at least a portion of the inner liner.

Alternatively or additionally to any of the examples above, wherein the support member is positioned between the inner liner and the sheath.

Alternatively or additionally to any of the examples above, wherein the plurality of ribs are free to move relative to the liner, the sheath, or both the liner and the sheath.

Alternatively or additionally to any of the examples above, wherein the plurality of ribs includes a first set of rib members and a second set of rib members, and wherein the first set of rib members interdigitate with the second set of rib members.

A method of manufacturing an implant delivery system includes:
coupling an implantable heart valve to a distal end region of a catheter shaft, wherein the heart valve is releasable from the catheter shaft;
inserting an expandable introducer sheath over a proximal end region of the catheter shaft;
attaching a handle to the proximal end region of the catheter shaft.

The above summary of some examples is not intended to describe each disclosed embodiment or every implementation of the present disclosure. The Figures, and Detailed Description, which follow, more particularly exemplify these examples.

### BRIEF DESCRIPTION OF FIG.S

FIG. 1 is a perspective view of an example introducer system;
FIG. 2 is a perspective view of an example introducer;
FIG. 3 is a cross-sectional view of an example introducer;
FIG. 4 is a cross-sectional view of an example introducer;
FIG. 5 is a perspective view of an example ribcage;
FIG. 6 is a perspective view of an example ribcage;
FIGS. 7-10 illustrate an example medical device being withdrawn through an example introducer.

While the disclosure is amenable to various modifications and alternative forms, specifics thereof have been shown by way of example in the drawings and will be described in detail. It should be understood, however, that the intention is not to limit the disclosure to the particular examples described. On the contrary, the intention is to cover all modifications, equivalents, and alternatives falling within the scope of the disclosure.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some examples", "other examples", etc., indicate that the embodiment described may include one or more particular features, structures, and/or characteristics. However, such recitations do not necessarily mean that all examples include the particular features, structures, and/or characteristics. Additionally, when particular features, structures, and/or characteristics are described in connection with one embodiment, it should be understood that such features, structures, and/or characteristics may also be used connection with other examples whether or not explicitly described unless clearly stated to the contrary.

The following detailed description should be read with reference to the drawings in which similar elements in different drawings are numbered the same. The drawings, which are not necessarily to scale, depict illustrative examples and are not intended to limit the scope of the disclosure.

Diseases and/or medical conditions that impact the cardiovascular system are prevalent throughout the world. Traditionally, treatment of the cardiovascular system was often conducted by directly accessing the impacted part of the body. For example, treatment of a blockage in one or more of the coronary arteries was traditionally treated using coronary artery bypass surgery. As can be readily appreciated, such therapies are rather invasive to the patient and require significant recovery times and/or treatments. More recently, less invasive therapies have been developed. For example, therapies have been developed which allow a blocked coronary artery to be accessed and treated via a percutaneous catheter (e.g., angioplasty). Such therapies have gained wide acceptance among patients and clinicians.

Some relatively common medical conditions may include or be the result of inefficiency, ineffectiveness, or complete failure of one or more of the valves within the heart. For example, failure of the aortic valve or the mitral valve can have a serious effect on a human and could lead to serious health condition and/or death if not dealt with properly. Treatment of defective heart valves poses other challenges in that the treatment often requires the repair or outright replacement of the defective valve. Such therapies may be highly invasive to the patient. Disclosed herein are medical devices that may be used for delivering a medical device to a portion of the cardiovascular system in order to diagnose, treat, and/or repair the system. At least some of the medical devices disclosed herein may be used to deliver and implant a replacement heart valve (e.g., a replacement aortic valve, replacement mitral valve, etc.). In addition, the devices disclosed herein may deliver the replacement heart valve percutaneously and, thus, may be much less invasive to the patient. The devices disclosed herein may also provide a number of additional desirable features and benefits as described in more detail below.

As discussed above, inserting a medical device (e.g., an implantable heart valve) into the vasculature may result in undesirable forces being applied to the vessel walls. As the medical device passes into the vasculature, it may make undesirable contact with one or more vessel walls. This interference may cause injury to the vessel as the medical device is navigated into calcified or diseased vessels. Therefore, in some instances an introducer system is utilized to position an introducer sheath into the vessel, whereby the introducer sheath is utilized to facilitate the insertion of medical device (e.g., an implantable heart valve) into the vessel. For example, in some instances it may be desirable to dispose an introducer sheath along the shaft of a medical device delivery system (e.g., an implantable heart valve delivery system), whereby the distal portion of the delivery system is withdrawn through the expandable introducer sheath as the system is retracted out of the body. This configuration permits the introducer sheath to remain in an unexpanded configuration as the delivery system is advanced into a body lumen, while also permitting the introducer sheath to expanded in the body lumen after the delivery system has been withdrawn back through the introducer sheath. The expanded introducer sheath may then be utilized to advance other medical devices therethrough. The following examples disclose an intravascular medical device including an expandable introducer, whereby the introducer is designed to expand from a reduced profile, unexpanded configuration to an expanded configuration.

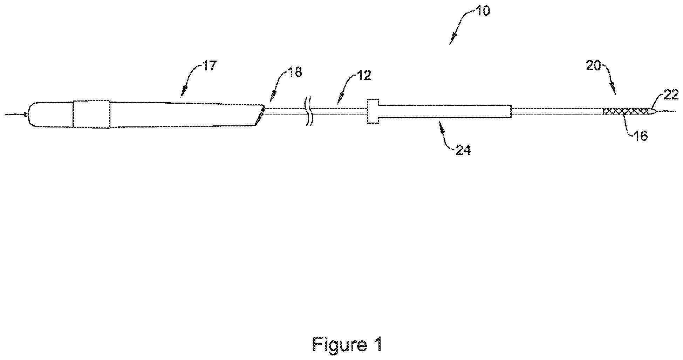

The figures illustrate selected components and/or arrangements of a medical device system 10, shown schematically in FIG. 1 for example. It should be noted that in any given figure, some features of the medical device system 10 may not be shown, or may be shown schematically, for simplicity. Additional details regarding some of the components of the medical device system 10 may be illustrated in other figures in greater detail. A medical device system 10 may be used to deliver and/or deploy a variety of medical devices to a number of locations within the anatomy. In at least some embodiments, the medical device system 10 may include a replacement heart valve delivery system (e.g., a replacement aortic valve delivery system) that can be used for percutaneous delivery of a medical implant 16, such as a replacement/prosthetic heart valve. This, however, is not intended to be limiting as the medical device system 10 may also be used for other interventions including valve repair, valvuloplasty, delivery of an implantable medical device (e.g., such as a stent, graft, etc.), and the like, or other similar interventions.

The medical device system 10 may generally be described as a catheter system that includes a shaft 12, an inner catheter (not shown) extending at least partially through a lumen of the shaft 12, and a medical implant 16 (e.g., a replacement heart valve implant) which may be coupled to the inner catheter and disposed within a lumen of the shaft 12 during delivery of the medical implant 16. The medical implant 16 may be disposed along a distal portion 20 of the shaft 12.

In some embodiments, a medical device handle 17 may be disposed at a proximal end 18 of the shaft 12 and/or the inner catheter and may include one or more actuation mechanisms associated therewith. In other words, one or more tubular members (e.g., the shaft 12, the inner catheter, etc.) may extend distally from the medical device handle 17. In general, the medical device handle 17 may be designed to manipulate the position of the shaft 12 relative to the inner catheter and/or aid in the deployment of the medical implant 16.

In use, the medical device system 10 may be advanced percutaneously through the vasculature to a position adjacent to an area of interest and/or a treatment location. For example, in some embodiments, the medical device system 10 may be advanced through the vasculature to a position adjacent to a defective native valve (e.g., aortic valve, mitral valve, etc.). Alternative approaches to treat a defective aortic valve and/or other heart valve(s) are also contemplated with the medical device system 10. During delivery, the medical implant 16 may be generally disposed in an elongated and low profile "delivery" configuration within the lumen and/or a distal end of the shaft 12, as seen schematically in FIG. 1, for example. Once positioned, the shaft 12 may be retracted relative to the medical implant 16 and/or the inner catheter to expose the medical implant 16. In some instances, the medical implant 16 may be self-expanding such that exposure of the medical implant 16 may deploy the medical implant 16. Alternatively, the medical implant 16 may be expanded/deployed using the medical device handle 17 in order to translate the medical implant 16 into a generally shortened and larger profile "deployed" configuration suitable for implantation within the anatomy. When the medical implant 16 is suitably deployed within the anatomy, the medical device system 10 may be disconnected, detached, and/or released from the medical implant 16 and the medical device system 10 can be removed from the vasculature, leaving the medical implant 16 in place in a "released" configuration.

It can be appreciated that during delivery and/or deployment of an implantable medical device (e.g., the medical implant 16), portions of the medical device system (e.g., the medical device system 10) may be required to be advanced through tortuous and/or narrow body lumens. Therefore, it may be desirable to utilize components and design medical delivery systems (e.g., such as the medical device system 10 and/or other medical devices) that minimize the profile of portions of the medical device that puncture (e.g., access) the body and advance through one or more body lumens. In other words, it may be beneficial to design medical device delivery systems such that they minimize the insertion site through which the medical device is advanced into a body lumen.

For example, as will be discussed in greater detail below, FIG. 1 illustrates that the medical device system 10 may include a tapered tip member 22. Tapered tip member 22 may be designed to be directly inserted through an insertion site on the surface of the body. In some instances, the insertion site may initially be formed by a clinician with a needle, through which a guidewire is advanced into the lumen of a vessel. After placement of the guidewire, medical device system 10 may be advanced over the guidewire, through the access site (e.g., puncture site) and directly into the body vessel. Once inside the vessel, medical device system 10 may be tracked to a treatment site.

However, in some instances it may be desirable to maintain a passageway (e.g., an access lumen) through which additional medical devices may be inserted into the vessel after the removal of medical device delivery system 10 from the body (e.g., after medical device delivery system has implanted the heart valve 16). Therefore, in some instances it may be desirable to position an introducer sheath through the access site and into the body vessel in order to maintain a passage through which other medical devices may be inserted into the body.

FIG. 1 shows an expandable introducer sheath 24 disposed along the outer surface of the shaft 12. It can be appreciated that the expandable introducer sheath 24 may be free to slide and/or rotate along the outer surface of shaft 12. While not shown in FIG. 1, it is further contemplated that the introducer sheath 24 may be engage and or mate with one or more tapered portions (not shown in FIG. 1) of shaft 12 and/or profiles of the distal end region 20 of the medical device system 10.

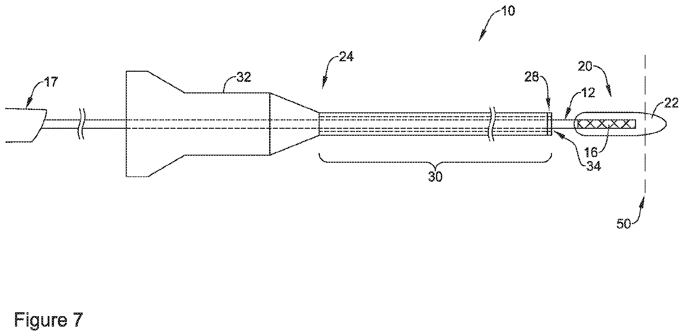

Additionally, FIG. 1 shows the introducer sheath 24 positioned between the handle 17 and the implantable medical device 16. The position of the introducer sheath 24 between handle 17 and the implantable medical device 16 may be referred to as an "in-line" or "pre-loaded" position of the introducer 24 on the medical device system 10. Further, it can be appreciated that having the introducer sheath 24 pre-loaded onto the shaft 12 may permit the tip member 22 to be inserted into a body lumen prior to the introducer sheath 24 being inserted into the body lumen. In other words, the tip member 22 itself may be initially advanced through a puncture site and into a body lumen, whereby the introducer sheath 24 may track over the shaft 12 and into the body lumen. FIGS. 7-10 further clarify this process.

Further, it can be appreciated that manufacturing the medical device system 10 shown in FIG. 1 may require an initial step of coupling the implantable medical device 16 to the distal end 20 of shaft 12. Next, the introducer sheath 24 may be advanced over the proximal end region 18 of the shaft 12. Finally, the handle member may be attached the proximal end region 18 of the shaft 12. In other examples, manufacturing the medical device system 10 shown in FIG. 1 may require an initial step of coupling the handle 17 to the proximal end 18 of shaft 12. Next, the introducer sheath 24 may be back-loaded over the distal end region 20 of the shaft 12. Finally, the implantable medical device 16 may be attached the distal end region 20 of the shaft 12.

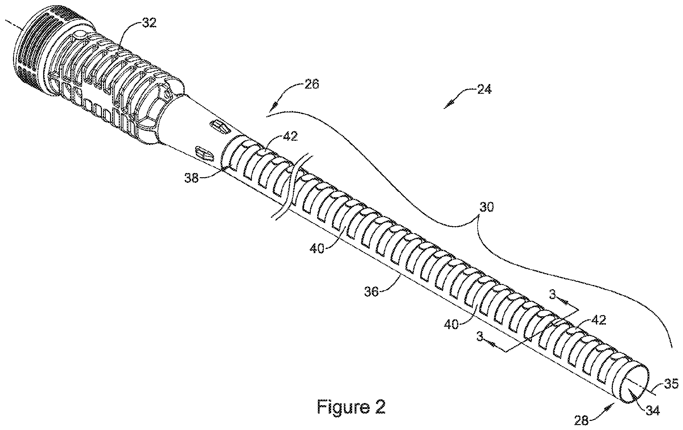

FIG. 2 is a perspective view of the introducer sheath 24 shown in FIG. 1. Introducer 24 may include a proximal end region 26, a distal end region 28 and an expandable portion 30. The proximal end region 26 of expandable portion 30 may be coupled to hub 32. Hub 32 may include an anti-splash back valve. Additionally, introducer 24 may further include an elongated compliant liner (not shown) having a lumen 34. The liner may extend along the expandable portion 30. The introducer 10 may also include an outer sheath 36. Outer sheath 36 may extend along (e.g., cover) expandable portion 30. As will be discussed in greater detail below, expandable section 30 may include a ribcage 38. Ribcage 38 may include a first set of rib members 40 positioned adjacent a second set of rib members 42.

As shown in FIG. 2, ribcage 38, the liner and the sheath 36 may extend the entire length of the expandable member 30. Additionally, ribcage 38 may shift from a first unexpanded configuration to and expanded configuration. For example, introducer 24 may shift from an unexpanded configuration to an expanded configuration. FIG. 2 further illustrates the curved shape of both first set of rib members 40 and second set of rib members 42 forms the curved shape of lumen 34.

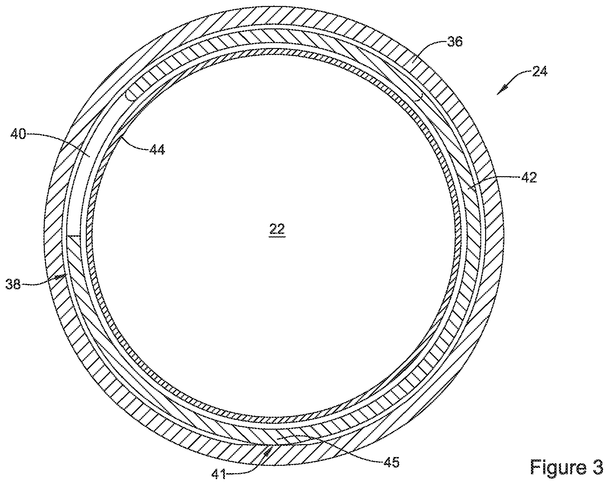

FIG. 3 shows a cross-sectional view along line 3-3 of FIG. 2. FIG. 3 depicts introducer 24 in an unexpanded configuration. Additionally, it can be appreciated that FIG. 3 represents a cross-section of the expandable member 30 of introducer 24. FIG. 3 illustrates a liner 44 (discussed above) extending along the inner surface of the expandable member 30. Further, FIG. 3 illustrates that a portion of ribcage 38 may attached (e.g., tacked, fused, integrated, engaged, etc.) with the tubular wall of outer sheath 36 at attachment location 41. In other words, FIG. 3 illustrates that in some examples, a portion of outer sheath 36 and a portion of ribcage 38 may be manufactured (e.g., tacked/fused/melted/reflowed) such that they are attached together. Further, it can be appreciated that a portion of ribcage 38 may be attached (e.g., tacked, fused, integrated, etc.) with the tubular wall of outer sheath 36 along the longitudinal axis 35 of introducer 24. In other words, it is contemplated that ribcage 38 may be attached to outer sheath 36 in a series of attachment locations 41 that are aligned with one another along the longitudinal axis 35 of introducer 24. However, this is not intended to be limiting. Rather, it is contemplated that ribcage 38 may be attached to outer sheath 36 in a variety of different locations along the interface of outer sheath 36 and ribcage 38.

As discussed above, ribcage 38 may include one or more first rib members 40 positioned adjacent to one or more second rib members 42. FIG. 3 illustrates spine 45 extending longitudinally along the longitudinal axis of introducer 24. In some examples, the attachment points 41 may be longitudinally aligned along the spine 45 of ribcage 38. However, this is not intended to be limiting. It is contemplated that in some examples, ribcage 38 is not attached to outer sheath 36. It is further contemplated that any portion of ribcage 38 (including spine 45) may be integral with all or a portion of outer sheath 36.

FIG. 3 further illustrates first rib member 40 and second rib member 42 extending away from spine 45 in a clockwise direction and counterclockwise direction, respectively. As shown in FIG. 3, both first rib member 40 and second rib member 42 include a curved portion which is positioned along the inner surface of sheath member 36, whereby the curved portion of first rib member 40 extends away from spine 45 in a first direction (e.g., clockwise direction) and the second rib member 42 extends away from the spine 45 in a second direction (e.g., counterclockwise direction).

An example methodology to construct the introducer 24 (shown in FIG. 1) may include configuring ribcage 38, outer sheath 36 and liner 44 as shown in FIG. 3 followed by applying heat such that outer sheath 36 member melts (e.g., reflows) around ribcage 38 and/or liner 44. It can be appreciated that liner 44 may be constructed from a material that has a higher melting point than either outer sheath 36 and/or ribcage 38, and therefore, will not melt upon the application of heat sufficient to melt outer sheath 36 and/or ribcage 38 together. In other examples, liner 44 may be constructed from a non-thermoplastic material designed to resist melting while heat is applied to reflow outer sheath 36 and ribcage 38 together. Additionally, it is contemplated that arrangement of liner 44, outer sheath 36 and ribcage 38 may include a variety of configurations throughout the manufacturing steps. For example, examples contemplated herein may include different positions, alignment, spacing, sizes, dimensions, etc. of ribcage 38, liner 44 and/or sheath 36 relative to one another during the manufacturing process and/or final design.

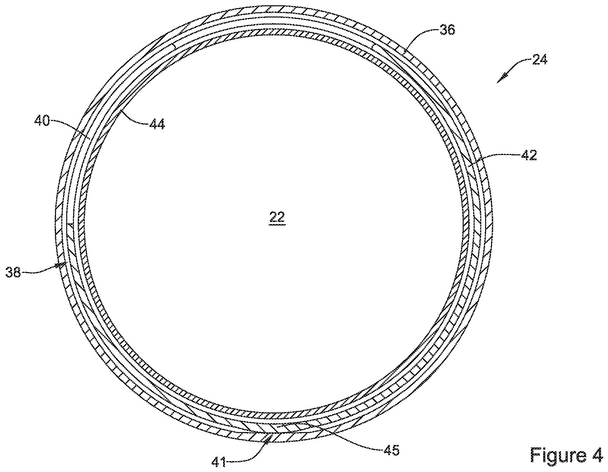

FIG. 4 is a cross-sectional view (along line 3-3 of FIG. 1) of the introducer 24 in an expanded configuration. For example, FIG. 4 may depict the expanded configuration of the unexpanded introducer illustrated in FIG. 3. In other words, FIG. 3 may depict the introducer 24 prior to a medical device being inserted and/or retracted through the expandable section 30 of the introducer 24, while FIG. 4 may depict the introducer 24 after a medical device has been inserted and/or retracted through the expandable member 30.

As shown in FIG. 4, the liner 44 has expanded radially outward. Additionally, it can be appreciated that the liner 44 may continue to expand after it initially contacts the inner surface of the ribcage 38, thereby applying a radially outward force on the first rib member 40 and the second rib member 42 of the ribcage 38.

However, as illustrated in FIG. 4 and described above, the first rib member 40 and the second rib member 42 of the ribcage 38 may expand (e.g., flex) radially outward to accommodate the outward expansion of the liner 44. FIG. 4 (in comparison to FIG. 3), illustrates the increased distance between the ends of the first rib member 40 and the second rib member 42. Specifically, in some examples it may be desirable to design the liner 44, the ribcage 38 and the sheath 36 to be able to move (e.g., slide) relative to one another as the expandable member 30 of the introducer 24 expands. For example, it may be desirable for the first rib member 40 and the second rib member 42 of the ribcage 38 to slide along the outer surface of liner 44. Additionally, as the liner 44 and the ribcage 38 are expanding, it is contemplated that the sheath 36 may slide along the outer surface of ribcage 38 and/or the liner 44. Further, in some instances a portion of the sheath member 36 may stretch to accommodate the expanded configuration (e.g., increased diameters) of both the liner 44 and/or the ribcage 38). It is contemplated that as the sheath 36 stretches, its wall thickness may decrease.

Additionally, can be appreciated that after a radially outward force is no longer applied to the sheath 36, the ribcage 38 and/or the liner 44, the introducer 24 may contract to a configuration that resembles the unexpanded configuration of the introducer 24 described above. It can be appreciated that after the introducer 24 has been expanded, its configuration may be different from its configuration pre-expansion. For example, after having been expanded, a portion of the liner 44 may extend beyond the first rib member 40 and the second rib member 42 of the ribcage 38. In other words, a portion of the liner 44 may be positioned between the outer surface of the first rib member 40 and/or the second rib member 42 and the inner surface of sheath 36.

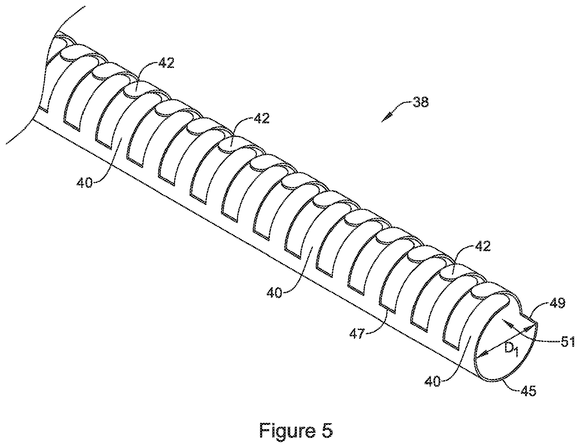

FIG. 5 illustrates an example ribcage 38. As shown in FIG. 5 and discussed above, ribcage 38 may include a first set of rib members 40 positioned adjacent (e.g., interdigitated, interlaced, etc.) a second set of rib members 42. Additionally, FIG. 5 illustrates that both the first set of rib members 40 and the second set of rib members 42 include a curved portion. Additionally, FIG. 5 further illustrates that the first set of rib members 40 and the second set of rib members 42 extend away from a central spine portion 45. Specifically, the first set of rib members 40 extend away from the central spine portion 45 in a clockwise direction while the second set of rib members 42 extend away from the central spine portion 45 in a counterclockwise direction. In some instances, first set of rib members 40 may be described as extending away from a first lateral edge 47 of central spine member 45 and the second set of rib members 42 may be described as extending away from a second lateral edge 49 of central spine member 45. Further, while first set of rib members 40 and second set of rib members 42 extend away from the first lateral edge 47 and second lateral edge 49 of spine member 45, they eventually begin to curve back toward and interdigitate with one another. As shown in FIG. 5, the curved shape of both first set of rib members 40 and second set of rib members 42 forms a lumen 51. Lumen 51 may include a diameter depicted as "D₁" in FIG. 5.

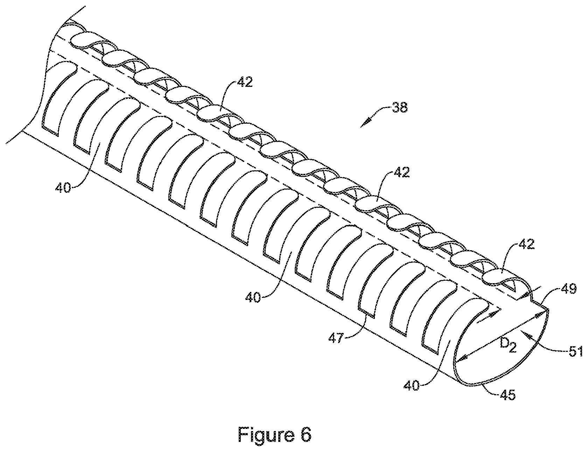

In some examples, ribcage 38 may be able to shift (e.g., radially expand) from a non-expanded configuration (such as that shown in FIG. 5) to an expandable configuration shown in FIG. 6. Specifically, the diameter of lumen 51 shown in FIG. 5 (depicted as diameter Di) may increase to define a larger diameter (depicted as "D₂" in FIG. 6). Further, FIG. 6 illustrates that as ribcage 38 expands, the first set of rib members 40 and the second set of rib members 42 may radially separate from one another. For example, FIG. 6 depicts the radial separation of the first set of rib members 40 from the second set of rib members 42 via a plurality of double-ended arrows. The tips of the double-ended arrows shown in FIG. 6 represent the radial separation between the ends of each of the first set of rib members 40 and the second set of rib members 42, respectively.

In contrast to the rib members described in above, in some examples it is contemplated that the first set of rib members 40 and second set of rib members 42 in FIG. 5 may not interdigitate with one another. Rather, each end of the first set of rib members 40 may radially align with each end of the second set of rib members 42. In other words, the ends of the first set of rib members 40 may not extend past (e.g., may not interdigitate with) the ends of the second set of rib members 42.

FIGS. 7-10 illustrate an example method of use of the medical device system 10 and introducer 24. As discussed above, in some instances, the introducer sheath 24 may be pre-loaded onto the shaft 12 of the medical device system 10. For example, FIG. 7 shows introducer 24 disposed along shaft 12 and positioned between the handle 17 and the implantable medical device 16.

FIG. 7 shows that in some instances the distal end region 20 of the medical device system 100 may include a tip member 22 which is designed to be advanced through an access site of a patient. For simplicity purposes, the dotted line 50 in FIG. 7 represents the access site (e.g., puncture site) into the body. Further, FIG. 7 shows that the tip member 22 being inserted through the access site and into the body. Tip member 22 may include one or more tapered regions to aid the insertion of the tip 22 into the body. FIG. 7 further illustrates that the implantable medical device 16, shaft 12 and the introducer sheath 24 (including expandable portion 30) have not been advanced into the body.

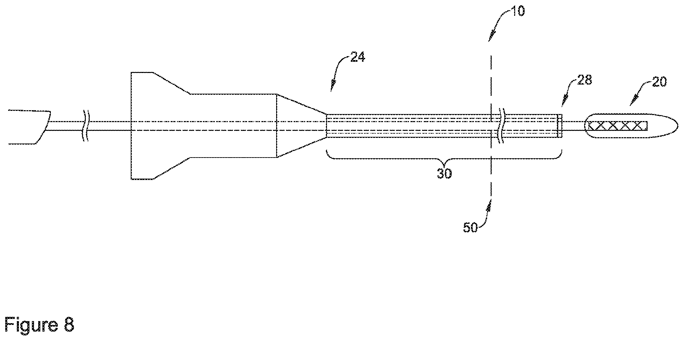

FIG. 8 illustrates the implantable medical device 16 having been inserted into the body (e.g., a body lumen). Additionally FIG. illustrates that the introducer sheath 24 has been advanced along the shaft 12 to a position in which the distal end region 28 of the shaft is positioned within the body (e.g., a body lumen). As illustrated in FIG. 8, the expandable portion 30 of the introducer sheath 24 may remain in an unexpanded configuration as the introducer sheath is being inserted in the body. Additionally, FIG. 8 shows that the implantable medical device 16 has not been deployed (e.g., released) from the example medical device delivery system 10.

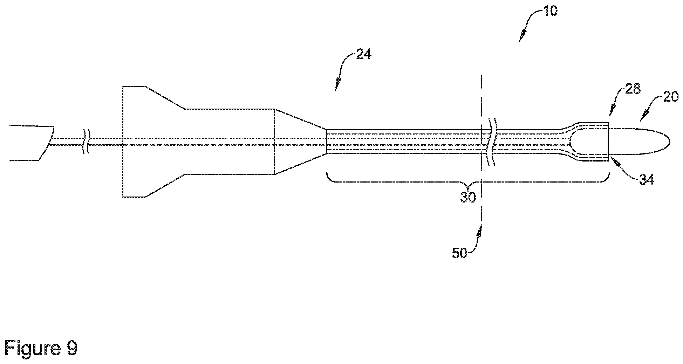

FIG. 9 illustrates the medical device system 10 being withdrawn from the body after the implantable medical device 16 (not shown in FIG. 9) has been deployed at a target site. FIG. 9 illustrates that the distal end portion 20 of the medical device system 10 has been withdrawn to a position in which it is contacting the distal end region 28 of the introducer sheath 24. As can be appreciated from FIG. 9 and the above discussion, the distal end region 28 of the introducer sheath 24 may expand radially outward as the medical device system 10 is withdrawn therethrough. In other words, FIG. 9 illustrates that the distal end region 28 may flare (e.g., expand) radially outward to accept the distal end region 20 of the medical device system 10.

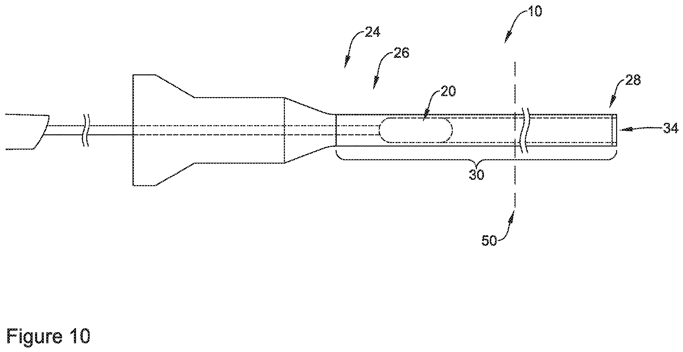

FIG. 10 illustrates the distal end region 20 of the medical device system 10 being withdrawn from the distal end region 28 to the proximal end region 26 of the introducer sheath 24. As described above, as the distal end region 20 of the medical device system 10 reaches, encounters, and/or engages the lumen 34 of the introducer 24, the medical device may exert a radially outward force from within the lumen 34 upon the wall of the expandable section 30 of the introducer sheath 24. The radially outward force may cause the expandable section 30 to expand as the distal end region 20 of the medical device 10 is retracted through the expandable section 30 of the introducer sheath 24. It can be appreciated from FIG. 10 that after the medical device system 10 has been removed from the introducer sheath 24, at least a portion of the introducer sheath 24 may remain inside the body in an expanded configuration. A clinician may then insert additional medical devices through the expanded introducer sheath 24.

It is contemplated that the expansion of the of the introducer sheath 24 from an unexpanded configuration to an expanded configuration may be variable. For example, the diameter of the unexpanded introducer sheath 24 may increase to an expanded diameter, after which, it may contract to a diameter that is greater than the diameter of the unexpanded configuration. However, this is not intended to be limiting. It is contemplated that once the unexpanded distal section is expanded, it may remain expanded or it may return to any diameter less than the expanded diameter (including a diameter that is less than the unexpanded diameter).

In some examples, introducer 24 (or other introducers and components thereof described herein) may be made from materials such as metals, metal alloys, polymers, ceramics, metal-polymer composites, or other suitable materials, and the like. Some examples of suitable materials may include metallic materials such as stainless steels (e.g. 304v stainless steel or 316L stainless steel), nickel-titanium alloys (e.g., nitinol, such as super elastic or linear elastic nitinol), nickel-chromium alloys, nickel-chromium-iron alloys, cobalt alloys, nickel, titanium, platinum, or alternatively, a polymeric material, such as a high performance polymer, or other suitable materials, and the like. The word nitinol was coined by a group of researchers at the United States Naval Ordinance Laboratory (NOL) who were the first to observe the shape memory behavior of this material. The word nitinol is an acronym including the chemical symbol for nickel (Ni), the chemical symbol for titanium (Ti), and an acronym identifying the Naval Ordinance Laboratory (NOL).

In some examples, the introducer 24 (or other introducers and components thereof described herein) may be made from materials such as, for example, a polymeric material, a ceramic, a metal, a metal alloy, a metal-polymer composite, or the like. Examples of suitable polymers may include polyurethane, a polyether-ester such as ARNITEL^{®} available from DSM Engineering Plastics, a polyester such as HYTREL^{®} available from DuPont, a linear low density polyethylene such as REXELL^{®}, a polyamide such as DURETHAN^{®} available from Bayer or CRISTAMID^{®} available from Elf Atochem, an elastomeric polyamide, a block polyamide/ether, a polyether block amide such as PEBA available under the trade name PEBAX^{®}, silicones, polyethylene, Marlex high-density polyethylene, polyetheretherketone (PEEK), polyimide (PI), and polyetherimide (PEI), a liquid crystal polymer (LCP) alone or blended with other materials. In some examples, a suitable polymeric material may have a yield strain of at least 20%, at least 30%, at least 40%, at least 50%, or more. In some examples, the sheath, the membrane, and/or the plurality of corrugations may be made from a material having a low coefficient of friction. In some examples, the sheath, the membrane, and/or the plurality of corrugations may be formed from a fluoropolymer, such as polytetrafluoroethylene (PTFE) or fluorinated ethylene propylene (FEP).

Portions of introducer 24 (or other introducers and components thereof described herein) may be made of, may be doped with, may include a layer of, or otherwise may include a radiopaque material. Radiopaque materials are understood to be materials capable of producing a relatively bright image on a fluoroscopy screen or another imaging technique such as X-ray during a medical procedure. This relatively bright image aids the user of device in determining its location. For example, one or more of the elements described above (i.e., the sheath, the membrane, the medical device, etc.) may include or be formed from a radiopaque material. Suitable materials can include, but are not limited to, bismuth subcarbonate, iodine, gold, platinum, palladium, tantalum, tungsten or tungsten alloy, and the like.

It should be understood that although the above discussion was focused on percutaneous medical procedures within the vasculature of a patient, other examples or methods in accordance with the disclosure can be adapted and configured for use in other parts of the anatomy of a patient. For example, devices and methods in accordance with the disclosure can be adapted for use in the digestive or gastrointestinal tract, such as in the mouth, throat, small and large intestine, colon, rectum, and the like. For another example, devices and methods can be adapted and configured for use within the respiratory tract, such as in the mouth, nose, throat, bronchial passages, nasal passages, lungs, and the like. Similarly, the devices and methods described herein with respect to percutaneous deployment may be used in other types of surgical procedures as appropriate. For example, in some examples, the devices may be deployed in a non-percutaneous procedure. Devices and methods in accordance with the disclosure can also be adapted and configured for other uses within the anatomy.

It should be understood that this disclosure is, in many respects, only illustrative. Changes may be made in details, particularly in matters of shape, size, and arrangement of steps without exceeding the scope of the disclosure. The disclosure's scope is, of course, defined in the language in which the appended claims are expressed.

## Claims

1. An implant delivery system (10), comprising:
a catheter shaft (12) having a distal end region (20) and a proximal end region (18);
an implant (16) coupled to the distal end region (20) of the catheter shaft (12);
a handle (17) coupled to the proximal end region (18) of the catheter shaft (12); and
an expandable introducer sheath (24) disposed along the catheter shaft (12) in an unexpanded position;
**characterized in that** the introducer sheath (24) is pre-loaded on the implant delivery system (10) and configured to shift from the unexpanded position to an expanded position as the distal end region of the catheter shaft is withdrawn through the introducer sheath (24).

2. The implant delivery system of claim 1, wherein the introducer sheath (24) is slidable along the catheter shaft (12).

3. The implant delivery system of any one of claims 1-2, wherein the distal end region (20) is designed to puncture into a body lumen.

4. The implant delivery system of any one of claims 1-3, wherein the introducer sheath (24) includes:
an inner liner (44) including a lumen (34);
an expandable support member (38), the support member (38) includes a plurality of ribs (40, 42) extending along a length of the support member (38); and
a sheath (36) attached to at least a portion of the support member (38);
wherein the support member (38) is designed to shift from the unexpanded position to the expanded position.

5. The implant delivery system of claim 4, wherein the plurality of rib members (40, 42) extend along only a portion of the introducer sheath (24).

6. The introducer of any one of claims 4-5, wherein the expandable support member (38) extends around at least a portion of the inner liner (44).

7. The introducer of any one of claims 4-6, wherein the support member (38) is positioned between the inner liner (44) and the sheath (36).

8. The introducer of any one of claims 4-7, wherein the plurality of ribs (40, 42) are free to move relative to the liner (44), the sheath (36), or both the liner and the sheath (36).

9. The introducer of any one of claims 4-8, wherein the plurality of ribs (40, 42) includes a first set of rib members (40) and a second set of rib members (42), and wherein the first set of rib members (40) interdigitate with the second set of rib members (42).

10. The introducer of any one of claims 1-9, wherein the implant is an implantable heart valve

## Patentansprüche

1. Implantatzuführungssystem (10), umfassend:
einen Katheterschaft (12) mit einem distalen Endbereich (20) und einem proximalen Endbereich (18),
ein Implantat (16), das an den distalen Endbereich (20) des Katheterschafts (12) gekoppelt ist,
einen Griff (17), der an den proximalen Endbereich (18) des Katheterschafts (12) gekoppelt ist, und
eine expandierbare Einführerhülse (24), die entlang des Katheterschafts (12) in einer nicht expandierten Position angeordnet ist,
**dadurch gekennzeichnet, dass** die Einführerhülse (24) an dem Implantatzuführungssystem (10) vorgeladen und dazu ausgestaltet ist, von der nicht expandierten Position zu einer expandierten Position zu wechseln, während der distale Endbereich des Katheterschafts durch die Einführerhülse (24) herausgezogen wird.

2. Implantatzuführungssystem nach Anspruch 1, wobei die Einführerhülse (24) entlang des Katheterschafts (12) verschiebbar ist.

3. Implantatzuführungssystem nach einem der Ansprüche 1 - 2, wobei der distale Endbereich (20) zum Einstechen in ein Körperlumen ausgelegt ist.

4. Implantatzuführungssystem nach einem der Ansprüche 1 - 3, wobei die Einführerhülse (24) Folgendes aufweist:
eine innere Auskleidung (44), die ein Lumen (34) aufweist,
ein expandierbares Stützglied (38), wobei das Stützglied (38) eine Vielzahl von Rippen (40, 42) aufweist, die sich entlang einer Länge des Stützglieds (38) erstrecken, und
eine an mindestens einem Abschnitt des Stützglieds (38) angebrachte Hülse (36),
wobei das Stützglied (38) zum Wechseln von der nicht expandierten Position zu der expandierten Position ausgelegt ist.

5. Implantatzuführungssystem nach Anspruch 4, wobei sich die Vielzahl von Rippengliedern (40, 42) nur entlang eines Abschnitts der Einführerhülse (24) erstrecken.

6. Einführer nach einem der Ansprüche 4 - 5, wobei sich das expandierbare Stützglied (38) um mindestens einen Abschnitt der inneren Auskleidung (44) erstreckt.

7. Einführer nach einem der Ansprüche 4 - 6, wobei das Stützglied (38) zwischen der inneren Auskleidung (44) und der Hülse (36) positioniert ist.

8. Einführer nach einem der Ansprüche 4 - 7, wobei sich die Vielzahl von Rippen (40, 42) frei bezüglich der Auskleidung (44) oder der Hülse (36) oder sowohl der Auskleidung als auch der Hülse 36 bewegen kann.

9. Einführer nach einem der Ansprüche 4 - 8, wobei die Vielzahl von Rippen (40, 42) einen ersten Satz Rippenglieder (40) und einen zweiten Satz Rippenglieder (42) aufweisen und wobei der erste Satz Rippenglieder (40) mit dem zweiten Satz Rippenglieder (42) verschränkt ist.

10. Einführer nach einem der Ansprüche 1 - 9, wobei das Implantat eine implantierbare Herzklappe ist.

## Revendications

1. Système de pose d'implant (10), comprenant :
une tige à cathéter (12) comportant une région d'extrémité distale (20) et une région d'extrémité proximale (18) ;
un implant (16) accouplé à la région d'extrémité distale (20) de la tige à cathéter (12) ;
un manche (17) accouplé à la région d'extrémité proximale (18) de la tige à cathéter (12) ; et
une gaine d'introduction élargissable (24) disposée le long de la tige à cathéter (12) dans une position non élargie ;
**caractérisé en ce que** la gaine d'introduction (24) est préchargée sur le système de pose d'implant (10) et conçue pour passer de la position non élargie à une position élargie à mesure que la région d'extrémité distale de la tige à cathéter est rétractée à travers la gaine d'introduction (24).

2. Système de pose d'implant selon la revendication 1, dans lequel la gaine d'introduction (24) est propre à coulisser le long de la tige à cathéter (12).

3. Système de pose d'implant selon l'une ou l'autre des revendications 1 et 2, dans lequel la région d'extrémité distale (20) est conçue pour pénétrer dans une lumière corporelle par perforation.

4. Système de pose d'implant selon l'une quelconque des revendications 1 à 3, dans lequel la gaine d'introduction (24) comprend :
un revêtement intérieur (44) comprenant une lumière (34) ;
un élément de support élargissable (38), l'élément de support (38) comprenant une pluralité de côtes (40, 42) s'étendant le long d'une longueur de l'élément de support (38) ; et
une gaine (36) attachée à au moins une partie de l'élément de support (38) ;
dans lequel l'élément de support (38) est conçu pour passer de la position non élargie à la position élargie.

5. Système de pose d'implant selon la revendication 4, dans lequel la pluralité d'éléments formant côtes (40, 42) s'étendent le long d'une partie seulement de la gaine d'introduction (24).

6. Dispositif d'introduction selon l'une ou l'autre des revendications 4 et 5, dans lequel l'élément de support élargissable (38) s'étend autour d'au moins une partie du revêtement intérieur (44).

7. Dispositif d'introduction selon l'une quelconque des revendications 4 à 6, dans lequel l'élément de support (38) est placé entre le revêtement intérieur (44) et la gaine (36).

8. Dispositif d'introduction selon l'une quelconque des revendications 4 à 7, dans lequel la pluralité de côtes (40, 42) sont libres de se déplacer relativement au revêtement (44), à la gaine (36), ou à la fois au revêtement et à la gaine (36).

9. Dispositif d'introduction selon l'une quelconque des revendications 4 à 8, dans lequel la pluralité de côtes (40, 42) comprennent un premier jeu d'éléments formant côtes (40) et un second jeu d'éléments formant côtes (42), et dans lequel le premier jeu d'éléments formant côtes (40) sont en interdigitation avec le second jeu d'éléments formant côtes (42).

10. Dispositif d'introduction selon l'une quelconque des revendications 1 à 9, dans lequel l'implant est une valve cardiaque implantable.
